# EUROPÄISCHE PATENTSCHRIFT

(11) **EP 2 349 493 B1**
(45) Veröffentlichungstag und Bekanntmachung des Hinweises auf die Patenterteilung: **06.07.2016**
(21) Anmeldenummer: 09736125.7
(22) Anmeldetag: 23.09.2009
(51) Int. Cl.: A61Q 15/00, A61K 8/26

(54) **ANTITRANSPIRANTZUBEREITUNGEN MIT HYDROTALCIT**
ANTIPERSPIRANT PREPARATIONS COMPRISING HYDROTALCITE
PRÉPARATION ANTITRANSPIRANTE COMPRENANT DE L'HYDROTALCITE

(30) Priorität: 22.10.2008 DE 102008052746
(43) Veröffentlichungstag der Anmeldung: 03.08.2011
(73) Patentinhaber: Beiersdorf AG, 20253 Hamburg (DE)
(72) Erfinder: MIERTSCH, Heike, 22459 Hamburg (DE); BIEL, Stefan, 21077 Hamburg (DE)
(86) Internationale Anmeldenummer: PCT/EP2009/006866
(87) Internationale Veröffentlichungsnummer: WO 2010/046010

(56) Entgegenhaltungen:
- WO-A1-01/08642
- JP-A- 3 190 811
- JP-A- 2000 143 442
- JP-A- 2003 286 146

## Beschreibung

Die Erfindung umfasst Antitranspirantzubereitungen mit Hydrotalcit.

Der Mensch besitzt zwei unterschiedliche Formen von Schweißdrüsen. Die ekkrinen Schweißdrüsen sondern hauptsächlich Salz und Wasser ab und tragen üblicherweise nicht zur Geruchsbildung bei. Für den Geruch sind die apokrinen Schweißdrüsen verantwortlich, die Fettsäuren, Cholesterine und andere Verbindungen ausscheiden. Diese Substanzen werden von Bakterien auf der Haut zersetzt, wobei die Abbauprodukte den für Schweiß typischen Geruch erzeugen.

Um den Schweißgeruch über einen längeren Zeitraum zu unterdrücken, ist der Einsatz kosmetischer Zubereitungen unerlässlich. Den üblichen kosmetischen Desodorantien liegen unterschiedliche Wirkprinzipien zugrunde, die auch kombiniert werden können: Zum einen werden Deowirkstoffe eingesetzt, die das Wachstum der den Schweißgeruch verursachenden Bakterien unterdrücken. Zu diesen keimhemmenden (bakteriostatischen) Mitteln zählen beispielsweise Triclosan, Chlorhexidin oder die natürlich vorkommenden Verbindungen wie Farnesol und Phenoxyethanol.

Zum anderen werden Antitranspirantien eingesetzt, welche die Schweißabsonderung durch Blockierung der Schweißdrüsenausgänge behindern. In den weitaus meisten Antitranspirantien kann durch Adstringentien - vorwiegend Aluminiumsalze wie Aluminiumhydroxychlorid (Aluchlorhydrat) oder Aluminium/Zirkoniumsalze - die Bildung des Schweißes reduziert werden.

Auch die Kombination von Adstringentien mit antimikrobiell wirksamen Stoffen in ein und derselben Zusammensetzung ist gebräuchlich. Ferner werden Parfümstoffe zur Überdeckung des Schweißgeruches eingesetzt.

Nachteilig bei der Verwendung von Aluminiumchlorohydrat ist beispielsweise, dass Rückstände die Kleidung in unschöner Weise verfärben können und der niedrige pH-Wert (sauer) der kosmetischen Zubereitung das biologische Gleichgewicht der Haut unvorteilhaft beeinflusst.

Bekannt und gebräuchlich sind neben den flüssigen Antitranspirantien wie Aerosole, Zerstäuber und Roll-on auch feste Zubereitungen, beispielsweise Deo-Stifte ("Sticks"), Puder, Pudersprays, Intimreinigungsmittel usw.

Bei den Antitranspirantien werden wasserhaltige und wasserfreie Formulierungen unterschieden. Bei den wasserhaltigen Produkten handelt es sich in der Regel um Emulsionen, aber auch hydroalkoholische Lösungen oder sogenannte Seifengelstifte sind auf dem Markt zu finden.
Bei den wasserfreien Produkten handelt es sich üblicherweise um Suspensionen, das heißt in einem Trägeröl ist der Antitranspirant-Wirkstoff in Pulverform suspendiert. Diese wasserfreien Suspensionen finden in flüssiger Form Anwendung in Aerosolen, sie können aber auch durch Verdicker zu pastösen bis festen Produkten formuliert werden. Da die Freisetzung des Antitranspirant-Wirkstoffes aus wasserfreien Formeln nicht so gut wie bei wässrigen Systemen ist, ist auch die Konzentration der AT-Wirkstoffe höher. Das führt zu einem verstärkten Einfluss auf die Stabilität der Formulierungen, insbesondere auf die Parfümstabilität. Im Vergleich zu wässrigen Produkten kann der Parfümeindruck bei wasserfreien Suspensionen weniger frisch sein. Daher sind die Formulierungsbestandteile so zu wählen, dass möglichst wenig Wechselwirkungen mit den Antitranspirantsalzen stattfinden können.

In der US 20060127336 und JP 03-190811 werden reine Deodorantzubereitungen beschrieben. In der US 20060127336 umfassen die Zubereitungen Aluminiumsilikate. Als Adsorbentien können den Zubereitungen u.a. Hydroltalcit zugesetzt werden. Antitranspirantwirkstoffe und deren Nachteile werden darin nicht genannt.

In der DE 102007059678 werden wasserfreie Antitranspirant-Stifte, enthaltend mindestens eine Lipid- oder Wachskomponente mit einem Schmelzpunkt > 30°C, mindestens ein unter Normalbedingungen flüssiges Öl, ausgewählt aus linearen Polydimethylsiloxanen mit 2 bis 50 Siloxaneinheiten, maximal 5 Gew.-% Wasser, bezogen auf die Gesamtzusammensetzung, und mindestens einen Antitranspirant-Wirkstoff.
Das lineare Polydimethylsiloxan mit 2 bis 50 Siloxaneinheiten ist in einer Gesamtmenge von 10-60 Gew.-%, besonders bevorzugt 20-50 Gew.-%, im Antitranspirant-Stift enthalten.

Die US 6024945 beschreibt die Verwendung von Hydrotalcit (Dialuminum-hexamagnesium-carbonat-hexadecahydroxid-tetrahydrat) in Aerosolen, die 1,1-Difluoroethane enthalten. Hydrotalcit verhindert die Reaktion zwischen Aluminiumchlorohydraten (ACH) und dem Difluoroethane, die zu toxischen Acetaldehyden und zur Korrosion der Aerosoldose führt. In der EP 1284128 werden AT-Stifte mit einem organischen oder anorganischen Material zur Verhinderung der Gelbfärbung der Stifte offenbart
EP 1515691 beschreibt die Verwendung von Alurninium-Zirkonium-Glycin-Chlorohydrate mit einem geringen Metall-Chlorid-Verhaltnis und einem stabilisierenden basischen Rohstoff zur Verbesserung der Stabilität und des Geruchs. Die Base kann u.a. inorganisch, unlöslich in Wasser, z. B. Magnesiumoxid. Calciumoxid, sein. Die Formulierung ist wasserfrei.
DE 102004014294 A1 beschreibt Antitranspirantzubereitungen umfassend neben Mischoxiden auch 2-alkylverzweigte Säuren und/oder deren Derivate, wobei die 2-alkylverzweigte Säure 4 bis 38 Kohlenstoffatome aufweist. Als Mischoxide können u.a. Hydrotalcite gewählt werden. Das Problem der Gelbfärbung von AT-zubereitungen wird darin jedoch nicht erwähnt.
WO 0108642 A1 offenbart Antitranspirantzubereitungen umfassend basische Aluminiumverbindungen sowie ein oder mehrere Additive, aus denen wiederum Hydrotalcit gewählt werden kann. Wünschenswert ist es eine Antitranspirantzubereitung bereit zu stellen, die die geschilderten Nachteile des Standes der Technik nicht aufweist. Insbesondere wäre es wünschenswert, wenn eine AT-Zubereitung die Bildung von weißen Rückständen vermindert oder verhindert und gleichzeitig ein angenehmes Hautgefühl sowie eine hohe Parfümstabilität aufweist.
Die Erfindung umfasst eine Antitranspirantzubereitung umfassend ein oder mehrere Antitranspirantwirkstoffe sowie Hydrotalcit (Dialuminum hexamagnesium-carbonat-hexadecahydroxid-tetrahydrat), wobei der Wasseranteil weniger als 5 Gew.%, bezogen auf die Gesamtmasse der Zubereitung, beträgt. Die erfindungsgemäße Zubereitung umfasst keine 2-alkylverzweigten Säuren und/oder deren Derivate, sofern die 2-alkylverzweigte Säure 4 bis 38 Kohlenstoffatome umfasst. Damit das Hydrotalcit als Säurefänger in der Formulierung fungieren kann, ist der Zusatz von Wasser notwendig. Jedoch ist der Zusatz an Wasser auf maximal 5 Gew.%. bezogen auf die Gesamtmasse des Stiftes begrenzt.
Zur Aktivierung des Hydrotalcit ist Wasser einzusetzen, in einem Verhältnis von Wasser zu Hydrotalcit von 10:1 bis 1:10. Als Antitranspirantwirkstoffe finden Adstringenzien Verwendung, vornehmlich AluminiumVerbindungen. Die früher eingesetzten, stark sauer wirkenden Salze Aluminiumsulfat od. -chlorid und die Agaricinsäure sind weitgehend durch Aluminiumhydroxychlorid u. -alkoholate ersetzt worden. Die nachfolgende Auflistung vorteilhaft einzusetzender Antitranspirant-Wirker soll in keinster Weise einschränkend sein:
Aluminium-Salze:
   - Aluminium-Salze wie Aluminiumchlorid AlCl₃, Aluminiumsulfat Al₂(SO₄)₃
   - Aluminiumchloride der empirischen Summenformel [Al₂(OH)ₘClₙ], wobei m+n=6
   - Aluminiumchlorhydrat [Al₂(OH)₅Cl] x H₂O
      o Standard Al-Komplexe: Locron P (Clariant), Micro-Dry (Reheis), ACH-331 (Summit), Aloxicoll PF 40 (Giulini).
      o Aktivierte Al-Komplexe: Reach 501 (Reheis), AACH-324 (Summit), AACH-7171 (Summit), Aloxicoll P (Giulini), Aloxicoll SD100
   - Aluminiumsesquichlorhydrat [Al₂(OH)_{4,5}Cl_{1,5}] x H₂O
      o Standard Al-Komplexe: Aluminum Sesquichlorohydrate (Reheis), AACH-308 (Summit)
      o Aktivierte Al-Komplexe: Reach 301 (Reheis)
   - Aluminiumdichlorhydrat [Al₂(OH)₄Cl₂] x H₂O
Aluminium-Zirkonium-Salze:
   - Aluminium/Zirkonium Trichlorhydrex Glycin [Al4Zr(OH)13Cl3] x H2O x Gly
      o Standard Al/Zr-Komplexe: Rezal 33GP (Reheis), AZG-7164 (Summit), Zirkonal P3G (Giulini)
      o Aktivierte Al/Zr-Komplexe: Reach AZZ 902 (Reheis), AAZG-7160 (Summit), Zirkonal AP3G (Giulini)
   - Aluminium/Zirkonium Tetrachlorhydrex Glycin [AI4Zr(OH)12CI4] x H2O x Gly
      o Standard Al/Zr-Komplexe: Rezal 36G (Reheis), AZG-368 (Summit), Zirkonal L435G (Giulini)
      o Aktivierte Al/Zr-Komplexe: Reach 908 (Reheis), AAZG-7167 (Summit), Zirkonal AP4G (Giulini)
   - Aluminium/Zirkonium Pentachlorhydrex Glycin [Al8Zr(OH)23Cl5] x H2O x Gly
   - Aluminium/Zirkonium Octachlorhydrex Glycin [Al8Zr(OH)20Cl8] x H2O x Gly

Ebenso von Vorteil können aber auch Glycin-freie Aluminium/Zirkonium-Salze sein.

Vorteilhaft kann auch die Verwendung von AT-Salz-Suspensionen bzw. -Gelen sein, bei denen pulverförmig vorliegende Aluminium-Salze in diversen Ölen dispergiert angeboten werden.

Die Antitranspirant-Wirkstoffe werden in den erfindungsgemäßen Formulierungen in einer Menge von 1 bis 35 Gew.%, vorzugsweise von 1 bis 25 Gew. %, eingesetzt.

Aluminiumsalze als AT-Wirkstoffe führen mitunter zu einem typischen saurem Geruch in Formulierungen. Dieser kann zwar durch Parfume überdeckt werden, tritt im Laufe der Lagerung jedoch zeitweilig wieder stärker auf. Des weiteren können Aluminiumsalze zu einer Gelbfärbung des Produktes führen, was vor allem bei pastösen und festen Produkten unerwünscht ist. Die Ursache dieser Geruchs- und Farbveränderungen könnte eine Reaktion der Aluminiumsalze mit Bestandteilen der Formulierung sein.

Diese Nachteile können erfindungsgemäß durch den Zusatz von Hydrotalcit in Kombination mit den geringen Mengen an Wasser vermindert bzw. vermieden werden.
Hydrotalcit ist ein internationaler Freiname für das als Antacidum wirksame Dialuminumhexamagnesium-carbonat-hexadecahydroxid-tetrahydrat, Al₂O₃ · 6MgO · CO₂ · 12H₂O bzw. Al₂Mg₆(OH)₁₆CO₃ · 4H₂O, *M*ᵣ 531,92. Hydrotalcit ist in Wasser praktisch unlöslich. Damit das Hydrotalcit aber als Säurefänger in der wasserarmen Formulierung fungieren kann, ist der Zusatz von Wasser notwendig. Hydrotalcit wurde 1970 von Kyowa patentiert und ist von Bayer (Talcid^{®}) und als Generikum im Handel erhältlich.
Hydrotalcit neutralisiert überschüssige Magensäure, die schädigend auf die Magenschleimhäute wirkt. Dadurch wird z.B. Sodbrennen gelindert und Magen-Darm-Geschwüren heilen schneller ab.
Unter Hydrotalcit werden erfindungsgemäß wasserhaltige Carbonate mit fremden Anionen verstanden. Besonders geeignet ist in diesem Sinne ein Aluminiumhydroxid-Magnesiumcarbonat, welches unter dem Handelsnamen Alma 3512 von SPI Pharma angeboten wird.
Des weiteren wird unter Hydrotalcit ein synthetisch hergestelltes Aluminium-Magnesium-Hydroxycarbonat verstanden, welches beispielsweise von SüdChemie angeboten und als Costabilisator bei der Herstellung von PVC und Polyolefinen verwendet wird.

Die Bezeichnung Hydrotalcit umfasst erfindungsgemäß die Verbindungen mit den Bezeichnungen Aluminiumhydroxid Magnesiumcarbonat und Dialuminum-hexamagnesiumcarbonat-hexadecahydroxid-tetrahydrat.

Hydrotalcit wird durch das aus den Antitranspirantwirkstoffen (AT-Wirker) freigesetzte freie Chlorid reduziert. Die AT-Formulierungen färben sich daraufhin nicht mehr gelb und die unangenehme Geruchsbildung wird vermieden. Dies führt zu einer besseren Parfümfreisetzung, einer besseren Parfümstabilität und zu weißen Produkten. Trotzdem Hydrotalcit das Produkt selbst weißer macht, werden die weißen Rückstände nicht verstärkt.
Hydrotalcit dient damit zur Vermeidung von Geruchs- und Farbveränderungen in Antitranspirantzubereitung umfassend ein oder mehrere Antitranspirant- und/oder Deodorantwirkstoffe und maximal 5 Gew.% Wasser, bezogen auf die Gesamtmasse der Zubereitung.
Auf den Zusatz von 2-alkylverzweigte Säuren und/oder deren Derivate, wobei die 2-alkylverzweigte Säure 4 bis 38 Kohlenstoffatome aufweisen, wird erfindungsgemäß verzichtet, da eine zusätzliche Wirksamkeit gegen Bakterien, Mycota, Schimmel und Viren nicht Aufgabe der vorliegenden Erfindung darstellt.
Der Zusatz an Wasser beträgt dabei maximal 5 Gew.% und definiert damit die oben erwähnte Obergrenze der "Wasserarmut" der erfindungsgemäßen AT-Stifte. Die Kombination von Hydrotalcit und Wasser liegt im Verhältnis von 10:1 bis 1:10.

Weitere mögliche und bevorzugte Bestandteile der Zubereitung sind mindestens ein Öl und mindestens ein Strukturgeber. Der Strukturgeber kann in Form von Suspendierhilfen für flüssige Produkte vorliegen oder auch als Verdicker (Fett bzw. Wachs) für pastöse bis feste Produkte.

Entsprechend der erfindungsgemäßen Verwendung können die erfindungsgemäßen Zubereitungen in verschiedenen Applikationsformen angeboten und konzipiert werden. Vorteilhaft liegen die AT- bzw. Desodorantzubereitungen in Form von Aerosolen, also in Aerosolbehältern, Quetschflaschen oder durch eine Pumpvorrichtung versprühbaren Präparate vor.

Ebenso ist aber auch eine Form von Roll-On-Vorrichtungen, als feste Deo-Stifte (Deo-Sticks) und in Form von aus normalen Flaschen und Behältern auftragbaren pastösen Formulierungen erfindungsgemäß. Weiterhin können die Zubereitungen vorteilhaft in Form von desodorierenden Tinkturen, desodorierenden Intimreinigungsmitteln, desodorierenden Shampoos, desodorierenden Dusch- oder Badezubereitungen, desodorierenden Pudern oder desodorierenden Pudersprays vorliegen.

Gemäß Stand der Technik umfassen wasserfreie bzw. wasserarme (< 5 Gew.%) Antitranspirantformulierungen:

Auf den Zusatz von 2-alkylverzweigte Säuren und/oder deren Derivate, wobei die 2-alkylverzweigte Säure 4 bis 38 Kohlenstoffatome aufweisen, wird erfindungsgemäß verzichtet, da eine zusätzliche Wirksamkeit gegen Bakterien, Mycota, Schimmel und Viren nicht Aufgabe der vorliegenden Erfindung darstellt.

Der Zusatz an Wasser beträgt dabei maximal 5 Gew.% und definiert damit die oben erwähnte Obergrenze der "Wasserarmut" der erfindungsgemäßen AT-Stifte

Die Kombination von Hydrotalcit und Wasser liegt im Verhältnis von 10:1 bis 1:10.

Weitere mögliche und bevorzugte Bestandteile der Zubereitung sind mindestens ein Öl und mindestens ein Strukturgeber. Der Strukturgeber kann in Form von Suspendierhilfen für flüssige Produkte vorliegen oder auch als Verdicker (Fett bzw. Wachs) für pastöse bis feste Produkte. Entsprechend der erfindungsgemäßen Verwendung können die erfindungsgemäßen Zubereitungen in verschiedenen Applikationsformen angeboten und konzipiert werden. Vorteilhaft liegen die AT- bzw. Desodorantzubereitungen in Form von Aerosolen, also in Aerosolbehältern, Quetschflaschen oder durch eine Pumpvorrichtung versprühbaren Präparate vor.
Ebenso ist aber auch eine Form von Roll-On-Vorrichtungen, als feste Deo-Stifte (Deo-Sticks) und in Form von aus normalen Flaschen und Behältern auftragbaren pastösen Formulierungen erfindungsgemäß. Weiterhin können die Zubereitungen vorteilhaft in Form von desodorierenden Tinkturen, desodorierenden Intimreinigungsmitteln, desodorierenden Shampoos, desodorierenden Dusch- oder Badezubereitungen, desodorierenden Pudern oder desodorierenden Pudersprays vorliegen

Gemäß Stand der Technik umfassen wasserfreie bzw. wässerarme (< 5 Gew.%) Antitranspirantformulierungen:

In wasserfreien Formulierungen wird neben dem leicht flüchtigen Trägeröl häufig mindestens ein nicht flüchtiges Öl, auch als Emollient bezeichnet, eingesetzt. Emollients dienen zur Verbesserung des Hautgefühls und können wesentlich dazu beitragen, weiße Rückstände zu vermeiden. Emollients, die zur Rückstandsminimierung beitragen, zeichnen sich durch einen relativ hohen Brechungsindex (>1,40) aus und werden auch als Maskierungsöle bezeichnet.

Nicht flüchtige Öle stammen bevorzugt aus der Gruppe der verzweigten und unverzweigten Kohlenwasserstoffe, der Silikonöle, der Dialkylether, der Dialkylcarbonate, der Gruppe der gesättigten oder ungesättigten, verzweigten Alkohole, sowie der Fettsäuretriglyceride, namentlich der synthetischen oder natürlichen Triglycerinester gesättigter und/oder ungesättigter, verzweigter und/oder unverzweigter Alkancarbonsäuren einer Kettenlänge von 8 bis 24, der Gruppe der Ester aus gesättigten und/oder ungesättigten, verzweigten und/oder unverzweigten Alkanmono- oder Dicarbonsäuren einer Kettenlänge von 1 bis 44 C-Atomen und gesättigten und/oder ungesättigten, verzweigten und/oder unverzweigten Alkoholen oder Diolen einer Kettenlänge von 1 bis 44 C-Atomen, aus der Gruppe der Ester oder Diester aus aromatischen und/oder nicht aromatischen Carbonsäuren und gesättigten und/oder ungesättigten, verzweigten und/oder unverzweigten Alkoholen (einwertig oder mehrwertig) einer Kettenlänge von 1 bis 30 C-Atomen, sofern die Ölkomponente oder die Gesamtheit der Öl-komponenten bei Raumtemperatur eine Flüssigkeit darstellen.

Strukturgeber können zum einen die Viskosität einer Formulierung erhöhen und zum anderen dazu dienen, bestimmte Rohstoffe wie Partikel in der Formulierung zu stabilisieren. Es wird in Verdicker und Suspendierhilfen unterschieden.

Unter Verdicker sind solche Stoffe zu verstehen, die bei einer Temperatur oberhalb der Raumtemperatur (20°C) schmelzen und beim Erstarren so kristallieren, dass die Viskosität der Formulierung erhöht wird und ein halbfestes bis festes Produkt entstehen kann. Insbesondere sind Fette und Wachse gemeint. Sie sind vorzugsweise in Wasser nicht löslich bzw. mit Wasser im aufgeschmolzenen Zustand nicht mischbar.

Fette sind bei 20°C feste oder halbfeste Stoffe des Pflanzen- oder Tierkörpers, die chemisch im wesentlichen aus gemischten Triglyceriden höherer Fettsäuren mit gerader Anzahl von Kohlenstoff-Atomen sowie geringen Anteilen von Acyllipiden (z.B. Sterolester) und dem Unverseifbaren bestehen. Viele Fremdbestandteile wie Mineralöle, Weichmacher und Biozide, die sich aufgrund ihres lipophilen Charakters im Fett anreichern, liegen ebenfalls im Unverseifbaren vor. Zusammen mit den Ölen, die chemisch ähnlich, jedoch bei 20°C flüssig sind, werden die Fette zur Gruppe der Lipide zusammengefasst.

Die Fette gehören häufig zu den niedrigschmelzenden Verdickern. Niedrigschmelzende Verdicker sind solche, dessen Schmelztemperatur unterhalb von 50°C liegt. Für Antitranspirantformulierungen sind besonders Verdicker bevorzugt, die im Bereich zwischen 30°C und 45°C schmelzen. Sie werden vorzugsweise aus der Gruppe der Triglyceride und/oder Ester ausgewählt.

Als hochschmelzende Verdicker sind Komponenten zu verstehen, die oberhalb von 50°C schmelzen. Besonders gut geeignet sind Wachse aller Art. Wachse haben in der Regel folgende Eigenschaften: bei 20°C knetbar, fest bis brüchig hart, grob bis feinkristallin, durchscheinend bis opak, jedoch nicht glasartig; über 40°C ohne Zersetzung schmelzend, schon wenig oberhalb des Schmelzpunktes verhältnismäßig niedrigviskos und nicht fadenziehend, stark temperaturabhängige Konsistenz und Löslichkeit, unter leichtem Druck polierbar. Sie unterscheiden sich von ähnlichen synthetischen oder natürlichen Produkten (z.B. Harzen, plastischen Massen, Metallseifen usw.) hauptsächlich darin, dass sie in der Regel etwa zwischen 50 und 90°C in den schmelzflüssigen, niedrigviskosen Zustand übergehen. Wachse können natürlichen Ursprungs, chemisch modifiziert oder synthetischen Ursprungs sein. Bei den natürlichen Wachsen wird nach pflanzlichen (z. B. Carnaubawachs, Candelillawachs), tierischen (z. B. Bienenwachs) und mineralischem Ursprung (z. B. Mikrowachse, Ceresin, Ozokerite) unterschieden.

Mikrowachse oder auch mikrokristalline Wachse sind höher schmelzende Inhaltsstoffe des Erdöls. Sie bestehen aus einem Gemisch gesättigter Kohlenwasserstoffe und enthalten noch Alkyl-substituierte Cycloparaffine und Alkyl-substituierte bzw. Naphthen-substituierte Aromaten. Im Gegensatz zu grobkristallinen Handelsparaffin weist Mikrowachs eine sehr feine Kristallstruktur auf.

In wasserfreien Antitranspirantformulierungen eignen sich als festigende Fett- oder Wachskomponente besonders mehr oder weniger alle gesättigten, linearen Fettsäuren und Fettalkohole mit 14-22 C-Atomen, Triglyceride gesättigter Fettsäuren mit 14-22 C-Atomen, gesättigte lineare Wachsester (Fettsäure-Fettalkohol-Ester) mit 28-44 C-Atomen, Wachse natürlichen Ursprungs, mikrokristalline Wachse oder Gemische davon.

Bevorzugt werden erfindungsgemäß mikrokristalline Wachse, wie Ceresinwachse, und Wachsester aus der Gruppe der Fettalkohole und Fettsäuren mit 28 bis 44 C-Atomen gewählt.

Geeignete Verdicker sind auch synthetische Wachse, wie das synthetisches Wachs, Fischer-Tropsch-Wachs der Fa. Sasol Sasolwax C80.

Entsprechend der DE 102007059678, in der das Vorhandensein flüchtiger Silikonöle, insbesondere Cyclomethicon ausgeschlossen sind, umfasst die erfindungsgemäße Stiftzubereitung kein lineares Polydimethylsiloxan mit 2 bis 50 Siloxaneinheiten.

Suspendierhilfen erhöhen die Stabilität von Partikeln in Suspensionen. In wasserfreien Antitranspirantformulierungen werden vorzugsweise modifizierte Schichtsilikate, Tonmineralien und/oder Kieselsäuren eingesetzt.

Vorteilhafte modifizierte Schichtsilikate im Sinne der vorliegenden Erfindung sind beispielsweise modifizierte Smektite. Smektite sind sehr feinkörnige (meist <2 mm), überwiegend als lamellenförmige, moosartige oder kugelförmige Aggregate vorkommende Dreischicht-Tonminerale, in denen eine zentrale Schicht aus oktaedrisch koordinierten Kationen sandwichartig von zwei Schichten aus [(Si,Al)O4]-Tetraedern umgeben ist. Vorteilhafte modifizierte Smektite sind z. B. modifizierte Montmorillonite. Montmorillonite werden durch die angenäherte chemische Formel Al2[(OH)2/Si4O10] • n H2O bzw. Al2O3 • 4 SiO2 • H2O • n H2O beschrieben und stellen zu den dioktaedrischen Smektiten gehörende Tonmineralien dar. Besonders vorteilhaft im Sinne der vorliegenden Erfindung sind ferner beispielsweise modifizierte Hektorite. Hektorite gehören zu den Smektiten und haben die angenäherte chemische Formel M+0,3(Mg2,7Li0,3)[Si4O10(OH)2], worin M+ meist Na+ darstellt.

Vorteilhaft im Sinne der vorliegenden Erfindung sind ferner modifizierte Bentonite. Bentonite sind Tone und Gesteine, die Smektite, vor allem Montmorillonit, als Hauptminerale enthalten. Die "Roh"-Bentonite sind entweder Calcium-Bentonite (in Großbritannien als Fuller-Erden bezeichnet) oder Natrium-Bentonite (auch: Wyoming-Bentonite).

Modifizierte Schichtsilikate im Sinne der vorliegenden Erfindung sind Schichtsilikate, insbesondere die bereits genannten Schichtsilikattypen, deren Organophilie (auch: Lipophilie) - z. B. durch Umsetzung mit quarternären Ammonium-Verbindungen - erhöht wurde. Solche Schichtsilikate werden auch als organophile Schichtsilikate bezeichnet.

Erfindungsgemäß besonders bevorzugt sind Stearalkoniumhektorit, ein Reaktionsprodukt aus Hektorit und Stearalkoniumchlorid (Benzyldimethylstearylammoniumchlorid), und Quaternium-18 Hektorit, ein Reaktionsprodukt aus Hektorit und Quaternium-18. Ebenso bevorzugt ist erfindungsgemäß Quaternium-90 Bentonite, ein Reaktionsprodukt aus Bentonite und Quaternium-90.

Bei der Verwendung von Tonmineralien kann zusätzlich ein sogenannter Aktivator verwendet werden. Diesem kommt die Aufgabe zu, das eingesetzte Tonmineral zu delaminieren, was auch als Aktivierung bezeichnet wird. Üblicherweise werden hierzu kleine, polare Moleküle wie Propylenglycolcarbonat und Ethanol eingesetzt, die sich unter mechanischem Energieeintrag zwischen die Schichten der Tonminerallamellen schieben und somit den gewünschten Vorgang durch elektrostatische Wechselwirkung mit diesen ermöglichen. Darüber hinaus bilden sie Wasserstoffbrückenbindungen zu den delamellierten Tonmineralplättchen aus und sorgen durch diese Brückenfunktion für den Zusammenhalt der entstehenden spielkartenhausähnlichen Struktur. Typischerweise zeigen diese Systeme eine stark ausgeprägte Thixotropie.

Kieselsäuren sind Verbindungen der allgemeinen Formel (SiO2)m • n H2O. Erfindungsgemäß haben die pyrogenen Kieselsäuren eine große Bedeutung. Unter der Bezeichnung pyrogene Kieselsäuren werden hochdisperse Kieselsäuren zusammengefasst, die durch Flammenhydrolyse (Typ A) hergestellt werden. Sie besitzen an ihrer nahezu porenfreien Oberfläche deutlich weniger OH-Gruppen als Fällungs-Kieselsäuren. Wegen ihrer durch die Silanol-Gruppen bedingten Hydrophilie werden die synthetischen Kieselsäuren häufig einem chemischen Nachbehandlungsverfahren unterzogen, bei denen die OH-Gruppen z. B. mit organischen Chlorsilanen reagieren. Dadurch entstehen modifizierte, z. B. hydrophobe Oberflächen, welche die anwendungstechnischen Eigenschaften der Kieselsäuren wesentlich erweitern. Sie sind unter den Handelnamen Aerosil und Cab-O-Sil mit verschiedenen Eigenschaften erhältlich.

Die Gesamtmenge an einer oder mehreren Suspendierhilfen in den erfindungsgemäßen Formulierungen liegt vorteilhaft in dem Bereich von 0,05 bis 5,0 Gew.-%, bevorzugt von 0,1 bis 3,0 Gew.-%, bezogen auf die Gesamtmasse der Stifte.

Weitere Komponenten der erfindungsgemäßen Zubereitung können sein: grenzflächenaktive Substanzen (Emulgatoren), Füllstoffe, Deowirkstoffe, Parfüm und Treibgas.

Auch können neben Fettalkoholen weitere Bestandteile üblicher Kosmetika enthalten sein.

Zusätzlich und erfindungsgemäß bevorzugt umfassen die AT-Stifte Emulgatoren.

Emulgatoren sind Hilfsmittel zur Herstellung und zur Stabilisierung von Emulsionen, die auch als grenzflächenaktive Stoffe bzw. Tenside bezeichnet werden können und in der Regel als ölige bis wachsartige, aber auch pulverförmige Stoffe vorliegen. Emulgatoren setzen die Grenzflächenspannung zwischen den beiden Phasen herab und erreichen neben der Verringerung der Grenzflächenarbeit auch eine Stabilisierung der gebildeten Emulsion. Sie stabilisieren die gebildete Emulsion durch Grenzflächenfilme sowie durch Ausbildung sterischer oder elektrischer Barrieren, wodurch das Zusammenfließen (Koaleszenz) der emulgierten Teilchen verhindert wird.

Damit Verbindungen als Emulgatoren wirksam sein können, müssen sie eine bestimmte Molekülstruktur aufweisen. Strukturelles Kennzeichen solcher Verbindungen ist ihr amphiphiler Molekülaufbau. Das Molekül einer solchen Verbindung besitzt wenigstens eine Gruppe mit Affinität zu Substanzen starker Polarität (polare Gruppe) und wenigstens eine Gruppe mit Affinität zu unpolaren Substanzen (apolare Gruppe).

In wasserfreien Suspensionen werden die Emulgatoren zur verbesserten Abwaschbarkeit der Formulierung von der Haut eingesetzt. Die in der wasserfreien Formel vorteilhaft enthaltenen Strukturgeber können auf der Haut fühlbar wachsige Rückstände hinterlassen. Durch die Anwesenheit von polaren Gruppen an den eingesetzten Emulgatoren wird beim Abwaschen der Formulierung die Affinität zum Wasser erhöht und die Rückstände verschwinden. Dafür eignen sich bevorzugt nichtionische Emulgatoren.

Unter nichtionischen Emulgatoren werden grenzflächenaktive Substanzen verstanden, die in wäßriger Lösung keine Ionen bilden. Die Hydrophilie solcher nichtionischer Emulgatoren wird durch den Anteil der polaren Gruppen im Molekül erreicht. Zu den nichtionischen Emulgatoren gehören Fettalkohole (z. B. Cetyl- oder Stearylalkohol), partielle Fettsäureester mehrwertiger Alkohole mit gesättigten Fettsäuren (z. B. Glycerolmonostearat), partielle Fettsäureester mehrwertiger Alkohole mit ungesättigten Fettsäuren (z. B. Glycerolmonooleat, Pentaerythritolmonooleat), ferner Polyoxyethylenester von Fettsäuren (z. B. Polyoxyethylenstearat), Polymerisationsprodukte aus Ethylenoxid und Propylenoxid an Fettalkoholen (Fettalkoholpolyglycolether) oder Fettsäuren (Fettsäureethoxylate).

Die Emulgatoren werden in den erfindungsgemäßen Formulierungen in einer Menge von 0,1 bis 10 Gew.%, vorzugsweise von 0,2 Gew.% bis 5 Gew. %, eingesetzt.

Des weiteren können die Stifte erfindungsgemäß bevorzugt auch Füllstoffe enthalten.

Als Füllstoffe sind partikuläre Rohstoffe zu verstehen, die sich gegenüber den restlichen Formulierungsbestandteilen inert verhalten. Sie tragen im wesentlichen zum Hautgefühl bei, können aber auch Aussehen und Struktur der Formulierung beeinflussen. Einfache, neutrale Füllstoffe sind vorzugsweise Talkum und Kaolin, aber auch Polysaccharide wie Stärken und Cellulosen und deren Derivate sind als Füllstoffe geeignet.

Die Füllstoffe werden in den erfindungsgemäßen Formulierungen in einer Menge von 0,5 bis 25 Gew.%, vorzugsweise von 1 Gew.% bis 20 Gew. %, eingesetzt.

Vorteilhaft sind die erfindungsgemäßen Zubereitungen als feste Zubereitungen, beispielsweise Deo-Stifte ("Sticks") formuliert.

Die nachfolgenden Beispiele sollen die vorliegende Erfindung verdeutlichen, ohne sie einzuschränken. Alle Mengenangaben, Anteile und Prozentanteile sind, soweit nicht anders angegeben, auf das Gewicht und die Gesamtmenge bzw. auf das Gesamtgewicht der Zubereitungen bezogen. Die in den Beispielen angegebenen Gewichtsprozente sind Aktivgehalte.

**Beispiele**

| Stifte | Beispiel 1 | Beispiel 2 | Beispiel 3 |
|---|---|---|---|
| Gehärtetes Rizinusöl | 1,5 | | 1,5 |
| Stearylalkohol | 20 | 5 | 20 |
| Glycerylstearat SE | 0,5 | | |
| Sasolwax C80¹ | | 2 | |
| Ceresine Isco Care 126² | | 10 | |
| Cetylpalmitat | | 7,5 | |
| PPG-14 Butylether | 15 | | 15 |
| Cyclomethicon | 41 | 28,5 | 40 |
| Caprylic/Capric Triglycerid | | 25 | |
| Wasser | 0,5 | 0,5 | 1,5 |
| Aluminiumhydroxid Magnesiumcarbonat³ | 0,5 | 0,5 | 1 |
| Aluminium Zirkonium Tetrachlorohydrex GLY | 16 | 16 | 16 |
| Talkum | 4 | 4 | 4 |
| Parfum | 1 | 1 | 1 |

| Aerosole | Beispiel 4 | Beispiel 5 | Beispiel 6 |
|---|---|---|---|
| Cyclomethicon | 7,550 | 6,125 | 11,125 |
| Dimethicon | 0,450 | | 1,000 |
| C12-15 Alkylbenzoat | | 2,250 | |
| Disteardimonium Hectorit | 0,600 | 0,525 | 0,700 |
| Wasser | 0,075 | 0,050 | 0,100 |
| Aluminiumhydroxid Magnesiumcarbonat ³ | 0,075 | 0,050 | 0,075 |
| Aluminiumchlorhydrat | 5,250 | 5,000 | 6,000 |
| Parfüm | 1,000 | 1,000 | 1,000 |
| Treibgas (Mischung aus Propan, Butan und Isobutan) | 85,000 | 85,000 | 80,000 |

| | | | |
|---|---|---|---|
| 1: ein synthetisches Wachs, Fischer-Tropsch-Wachs der Fa. Sasol 2: mikrokristallines Wachs, Ceresinwachs der Fa. PARAMELT BV 3: Hydrotalcit, Alma 3512 von SPI Pharma | | | |

## Patentansprüche

1. Antitranspirantzubereitung umfassend ein oder mehrere Antitranspirantwirkstoffe sowie Hydrotalcit (Dialuminum-hexamagnesium-carbonat-hexadecahydroxid-tetrahydrat), wobei der Wasseranteil weniger als 5 Gew.%, bezogen auf die Gesamtmasse der Zubereitung, beträgt und die Zubereitung frei von 2-alkylverzweigten Säuren und/oder deren Derivate ist, sofern die 2-alkylverzweigte Säuren 4 bis 38 Kohlenstoffatome aufweisen **dadurch gekennzeichnet, dass** das Gewichtsverhältnis von Wasser zu Hydrotalcit von 10:1 bis 1:10 ewählt wird.

2. Antitranspirantzubereitung nach Anspruch umfassend zusätzlich ein oder mehrere Trägeröle, Verdicker, Suspendierhilfen und/oder Emulgatoren.

3. Zubereitung nach Anspruch 2, **dadurch gekennzeichnet, dass** als Trageröle flüchtige Silikonöle, als Verdicker mikrokristalline Wachse, als Suspendierhilfen Smektite, Hektorite, Bentonite und/oder Stearalkoniumhektorit, als Emulgatoren Cetyl- oder Stearylalkohol gewählt werden.

4. Zubereitung nach einem der vorstehenden Ansprüche, **dadurch gekennzeichnet, dass** kein lineares Polydimethylsiloxan mit 2 bis 50 Siloxaneinheiten in der Zubereitung enthalten ist.

5. Verwendung von Hydrotalcit zur Vermeidung von Geruchs- und Farbveränderungen in Antitranspirantzubereitung umfassend ein oder mehrere Antitranspirant- und/oder Deodorantwirkstoffe und maximal 5 Gew.% Wasser, bezogen auf die Gesamtmasse der Zubereitung, **dadurch gekennzeichnet, dass** das Gewichtsverhaltnis von Wasser zu Hydrotalcit von 10:1 bis 1:10 gewählt wird.

## Claims

1. Antiperspirant preparation comprising one or more antiperspirant active ingredients and hydrotalcite (dialuminium hexamagnesium carbonate hexadecahydroxide tetrahydrate), where the water fraction is less than 5% by weight, based on the total mass of the preparation, and the preparation is free from 2-alkyl-branched acids and/or derivatives thereof if the 2-alkyl-branched acids have 4 to 38 carbon atoms, **characterized in that** the weight ratio of water to hydrotalcite is selected from 10:1 to 1:10.

2. Antiperspirant preparation according to Claim, additionally comprising one or more carrier oils, thickeners, suspension aids and/or emulsifiers.

3. Preparation according to Claim 2, **characterized in that** the carrier oils selected are volatile silicone oils, the thickeners selected are microcrystalline waxes, the suspension aids selected are smectites, hectorites, bentonites and/or stearalkonium hectorite, and the emulsifiers selected are cetyl alcohol or stearyl alcohol.

4. Preparation according to one of the preceding claims, **characterized in that** no linear polydimethylsiloxane with 2 to 50 siloxane units is present in the preparation.

5. Use of hydrotalcite for avoiding changes in odour and colour in antiperspirant preparation comprising one or more antiperspirant and/or deodorant active ingredients and at most 5% by weight of water, based on the total mass of the preparation, **characterized in that** the weight ratio of water to hydrotalcite is selected from 10:1 to 1:10.

## Revendications

1. Préparation anti-transpirante comprenant un ou plusieurs agents actifs anti-transpirants et de l'hydrotalcite (tétrahydrate d'hexadécahydroxyde de carbonate de dialuminium-hexamagnésium), la proportion d'eau étant de moins de 5 % en poids, par rapport à la masse totale de la préparation, et la préparation étant exempte d'acides 2-alkylramifiés et/ou leurs dérivés, dans la mesure où les acides 2-alkylramifiés comprennent 4 à 38 atomes de carbone, **caractérisée en ce que** le rapport en poids entre l'eau et l'hydrotalcite est choisi de 10:1 à 1:10.

2. Préparation anti-transpirante selon la revendication, comprenant en outre une ou plusieurs huiles supports, un ou plusieurs épaississants, adjuvants de suspension-et/ou émulsifiants.

3. Préparation selon la revendication 2, **caractérisée en ce que** des huiles de silicone volatiles sont choisies en tant qu'huiles supports, des cires microcristallines en tant qu'épaississants, des smectites, des hectorites, des bentonites et/ou de l'hectorite de stéaralconium en tant qu'adjuvants de suspension, l'alcool cétylique ou stéarylique en tant qu'émulsifiants.

4. Préparation selon l'une quelconque des revendications précédentes, **caractérisée en ce qu'**aucun polydiméthylsiloxane linéaire de 2 à 50 unités siloxane n'est contenu dans la préparation.

5. Utilisation d'hydrotalcite pour éviter des modifications d'odeur et de couleur dans une préparation anti-transpirante comprenant un ou plusieurs agents actifs anti-transpirants et/ou désodorisants et au plus 5 % en poids d'eau, par rapport à la masse totale de la préparation, **caractérisée en ce que** le rapport en poids entre l'eau et l'hydrotalcite est choisi de 10:1 à 1:10.
